# EUROPEAN PATENT APPLICATION

(11) **EP 4 378 469 A1**
(43) Date of publication of application: **05.06.2024**
(21) Application number: 22210983.7
(22) Date of filing: 02.12.2022
(51) Int. Cl.: A61K 36/45, A61P 35/00

(54) **THE USE OF PROANTHOCYANIDIN FRACTIONS OBTAINED FROM LINGONBERRY LEAVES AND FRUITS FOR THE PREPARATION OF CANCER CELL SPHEROIDS AND THEIR EFFECTS IN CANCER CELLS**

(71) Applicant: Lietuvos sveikatos mokslu universitetas, 44307 Kaunas (LT)
(72) Inventor: Petrikaite, Vilma, 44307 Kaunas (LT); Vilkickyte , Gabriel, 44307 Kaunas (LT); Raudone , Lina, 44307 Kaunas (LT)
(74) Representative: Zaboliene, Reda

(57) **Abstract**

The present invention describes the method for the preparation of cell spheroid comprising contacting a cell suspension with the lingonberry proanthocyanidin fractions, wherein the lingonberry proanthocyanidin fractions are dissolved in water. fractions of proanthocyanidins with anti-cancer activity against melanoma, kidney and colorectal cancer cells are isolated from lingonberry fruits and leaves. Studies of lyophilized fractions of proanthocyanidins in three-dimensional cultures of human colorectal, renal, and melanoma cells have shown that these fractions stimulate spheroid formation. The prepared spheroids, incubated for 4 days or more with the produced fractions, grew more slowly and began to degrade during the study, so these proanthocyanidins have anticancer effects in cancer cell cultures. The proanthocyanidin fraction of lingonberry leaves and the proanthocyanidin fraction of lingonberry fruit were found to inhibit the migration of human colorectal, renal, and melanoma cells.

## Description

### FIELD OF THE INVENTION

The present invention relates to the use of proanthocyanidin fractions, in particular to the use of proanthocyanidin fractions obtained from lingonberry (*Vaccinium vitis idaea* L.) leaves and fruits, for the preparation of cancer cell spheroids and as anticancer agents and the method of the preparation of cell spheroids.

### BACKGROUND

Three-dimensional cultures are an increasingly applied *in vitro* model for testing substances (including medicinal compounds) before testing them in animals. Such models, compared to conventional two-dimensional models, e.g., monolayer cell cultures, are superior, as they better mimic *in vivo* conditions - they form a gradient of the concentration of substances, the formation of intercellular interactions that are more similar to those found naturally in a living organism. Various methods are used to form spheroids from cancer cells, such as "hanging drop", non-stick surface formation, microfluidic devices, etc.

US7052720B1 describes a spheroid-forming activity which is obtained by heat treating fetal calf serum. Introduced into cell culture, the substance or mixture so obtained causes cells to grow in three-dimensional cultures as opposed to monolayer. The difference is that the present invention uses the proanthocyanidin fractions of plant origin, in particular lingonberry leaves and fruit.

US10329527B2 discloses a spheroid forming culture container using a temperature-sensitive glycol chitosan derivative and a spheroid forming method using the same. In the disclosed spheroid forming culture container, a surface of a culturing space is coated with a glycol chitosan derivative having reversible sol-gel transition characteristic depending on temperature. The difference is that the method of forming spheroids in the present invention does not require the purchase of special containers and is therefore cheaper and simpler.

AU2013201685A1 provides use of polymeric proanthocyanidin compositions from a Croton species or *Calophyllum* species in methods for preventing cancer of bowel diseases. The difference is that in the present invention the proanthocyanidin fractions are isolated from lingonberry fruits and leaves and the anti-cancer effect of the fractions acts on a wider spectrum against melanoma, kidney and colorectal cancer cells. Patent application US2008260695 describes a composition comprising a proanthocyanidin extract from grape seeds. This composition inhibits the growth of colorectal, ovarian, breast, liver, and cervical cancer cells. The difference is that in the present invention the proanthocyanidin fractions are isolated from lingonberry fruits and leaves, and these fractions are specifically effective against colorectal, renal and melanoma cancer cells.

In these methods, not all cells form three-dimensional cultures, and some methods require complex manipulations or expensive materials/tools. In the present invention, upon the addition of the produced proanthocyanidin fractions to the cell culture medium, the spheroids form spontaneously without the use of complicated procedures. Whole plant extracts, as well as lingonberry leaves and fruit extracts, are the most used in research, but the specific anti-cancer effects of lingonberry proanthocyanidin fraction on cancer cell migration and its effects in three-dimensional cultures have not been studied to date. Thus, the effect of inhibiting the migration of human colorectal, renal and melanoma cells and promoting the breakdown of formed spheroids is new.

### SUMMARY OF THE INVENTION

In the present invention provides a method for the preparation of cell spheroid comprising contacting a cell suspension with the lingonberry proanthocyanidin fractions, wherein the lingonberry proanthocyanidin fractions are dissolved in water. The cell cultures are selected from three-dimensional cultures of human colorectal, renal, and melanoma cells.

Prepared cell spheroid comprises cancer cells which are selected from colorectal cancer cell line, the human malignant melanoma cell line, and the human renal adenocarcinoma cell line.

In additional embodiment the method provides producing cell spheroids by contacting a cell line with lingonberry proanthocyanidin fractions Said fractions of proanthocyanidins with anti-cancer activity against melanoma, kidney and colorectal cancer cells are isolated from lingonberry (*Vaccinium vitis-idaea* L.) fruits and leaves. The concentration of lingonberry leaves extract is from 10 µg/ml to 100 µg/ml, the concentration of lingoberry fruit extract is from 20 µg/ml to 100 µg/ml.

The lingonberry proanthocyanidin fractions are included in a multi-well plate in which cell spheroids are prepared. The lingoberry proanthocyanidin fractions comprising cell spheroids are used for the assessing the property of a cell selected from any of formation, growth, proliferation, migration, viability, disintegration.

The lingonberry proanthocyanin fractions are use in the prevention or treatment of cancerous condition which is selected from the group consisting of colorectal cancer, kidney cancer, melanoma and cancer metastasis that displays accelerated cell migration.

Studies of lyophilized fractions of proanthocyanidins in three-dimensional cultures of human colorectal, renal, and melanoma cells have shown that these fractions stimulate spheroid formation. The formed spheroids, incubated for 4 days or more with the produced fractions, grew more slowly and began to disintegrate during the study, so these proanthocyanidins have anticancer effects in three-dimensional cultures. The proanthocyanidins fraction of lingonberry leaves and the proanthocyanidin fraction of lingonberry fruit were found to inhibit the migration of human colorectal, renal, and melanoma cells.

The acetone fractions isolated are dominated by procyanidins type A and B. Procyanidins A1, A2, C1, B1, B2, B3 were identified (of which procyanidin A1 and A2 predominate, accounting for 70% and 60% of the total compounds in the acetone fractions of lingonberry leaves and fruit, respectively).

Proanthocyanidin extract is used in the prevention and treatment of cancer, where the cancer is selected from the group consisting of colorectal cancer, kidney cancer and melanoma.

### BRIEF DESCRIPTION OF DRAWINGS

Fig. 1. lingonberry (*Vaccinium vitis idaea L.*), → leaves and fruit of lingonberry raw material, → total extract obtained from the raw material, → proanthocyanidin fractions.
Fig. 2. Effect of lingonberry leaves and fruit proanthocyanidin fractions on HT-29 cell migration after 12, 24, and 36 h.
Fig. 3. Effect of lingonberry leaves and fruit proanthocyanidin fractions on IGR39, CaKi-1 and HT-29 cell migration by "Wound Healing" method, n = 3.
Fig. 4. Effect of lingonberry leaves and fruit proanthocyanidin fractions on the growth of IGR39, CaKi-1 and HT-29 cell spheroids, expressed as a percentage compared to the control group (unaffected by extracts).
Fig. 5. Effect of lingonberry fruit proanthocyanidin fractions on the formation of HT-29, CaKi-1 and IGR39 spheroids in ultra-low attachment plates.
Fig. 6. Effect of lingonberry fruit proanthocyanidin fractions on spontaneous formation of HT-29 spheroids.
Fig. 7. Effect of lingonberry leaves and fruit proanthocyanidin fractions on the viability of IGR39, CaKi-1 and HT-29 cell spheroids by MTT reduction method after 10 days of incubation, expressed as a percentage compared to the control group (unaffected by proanthocyanidin fractions).

### DETAILED DESCRIPTION OF THE INVENTION

**The plant**. Lingonberry is a widespread species in the boreal region found in northern Europe, Asia and North America. Lingonberry leaves and fruits accumulate various groups of bioactive compounds (phenolic acids, flavonoids, anthocyanins, triterpene acids, organic acids, sugars and their alcohols, glycosylated hydroquinones, proanthocyanidins), which have positive health effects [Lee et al., 2012; Lima et al., 2009; Ek et al., 2006, Hokkanen et al., 2009]. The raw materials of lingonberry fruits and leaves used for the research were collected in 2019. in September from various natural habitats in North-Eastern Lithuania (56°00'40.6"N 25°31'29.4"E, 55°59'20.6"N 25°25'48.2"E, 56°04'32.8"N 25°29'41.1"N, 55°35'45.1"N 26°07'53.7"E, 55°39'59.3"N 25°59'26.7"E (WGS)). The harvested lingonberry fruits were frozen and lyophilized at 0.01 mbar, -85°C, and the lingonberry leaves were dried at room temperature in a well-ventilated area away from direct sunlight.

### Extraction

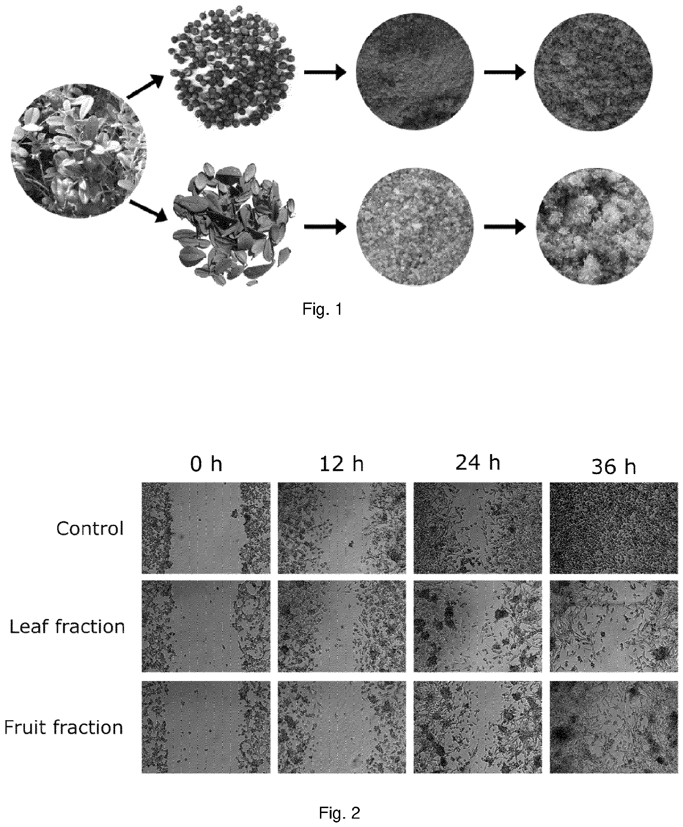

To obtain homogeneous mixtures, the samples from different areas were mixed well and ground with an electric grinder. The dry extracts of the lingonberry raw material used in the further fractionation were obtained by extracting the milled lingonberry fruits with acetone 70%, in a ratio of 1:15 (ultrasonic extraction was performed 3 times for 15 minutes at a frequency of 80 kWz and a power of 100 W), and extracting milled lingonberry leaves with 80 percent acetone in a ratio of 1:25 (ultrasonic extraction was performed 4 times for 15 min). The extracts were centrifuged at 10,500 rpm for 10 minutes at the rate of obtaining the precipitate with which further extraction has taken place, and the precipitate which has been combined and filtered through a paper filter at the end of all the extraction steps. The obtained liquid lingonberry extracts were concentrated by evaporation of the organic solvent acetone on a rotary evaporator at 40°C, and the remaining aqueous extract was lyophilized at 0.05 mbar to -50 ° C to obtain dry lingonberry fruit and lea extracts. The resulting lingonberry fruit and leaves powder was dissolved in 50 percent. The resulting solution was applied to a glass column (3 × 60 cm) packed with stationary phase dextran gel Sephadex LH-20 (swollen for 24 hours in 50% methanol in a ratio of 1: 5). During the fractionation, compounds soluble in water and aqueous alcohol mixtures were removed from the extracts. During the fractionation of the lingonberry leaves, the column was washed with two column volumes of water to remove non-phenolic compounds, phenolic acids and arbutin from the lingonberry leaves, passing four column volumes of 50% through the column. ethanol - removes flavanols and most monomeric flavanols. Finally, wash the column with two column volumes of 70% acetone, the brown fraction of proanthocyanidins collected, used for further studies. During the fractionation of lingonberry fruits, non-phenolic compounds and sugars were removed by washing the column with one column volume of water, and further washing the column with four column volumes of 20% methanol acidified with 2% formic acid - removal of anthocyanins and phenolic acids and passing four column volumes of 50% through the column, ethanol - flavanols and most monomeric flavanols. Like lingonberry leaves, the proanthocyanidin fraction of lingonberry fruit was collected by eluting two column volumes of 70% acetone. After collecting the acetone fractions of lingonberry fruits and leaves, they were concentrated on a rotary evaporator and lyophilized under the conditions described above (Fig. 1).

### Preparation of dry extracts for use

Dry lingonberry leaves and fruit proanthocyanidin fractions are dissolved in cell culture medium at concentrations of 0,01 mg/ml to 0,10 mg/ml before use and filtered through a 0.22 micron filter. Can be stored in a refrigerator (5 ± 3 C) for up to 7 days.

### Analysis of extracts

Samples of lingonberry dry proanthocyanidin fractions were analysed by a validated ESC-DMD method using an ACE Super C18 reverse phase column (250 mm × 4.6 mm, particle size 3 µm) and a gradient elution of 0.1% trifluoroacetic acid in water (eluent A) and acetonitrile (eluent B). Flow rate 0.5 ml / min, injection volume 10 µL, column temperature 35 ° C. Gradient model: 0 min, 10 percent. B; 0-40 min, 30 percent B; 40-60 min, 70 percent B; 60-64 min, 90 percent B; 64-70 min, 10 percent B. Lyophilized samples were dissolved in ethanol at 70% prior to ESC-DMD analysis obtaining concentration of 1 mg/ml and filtered through 0.2 µm PVDF filters. Proanthocyanidins type A and B were found to predominate in the isolated acetone fractions based on the retention times and UV absorption spectra of the sample and standard compounds. Procyanidins A1, A2, C1, B1, B2, B3 were identified (of which procyanidin A1 and A2 predominate, accounting for 70% and 60% of the total compounds in the acetone fractions of lingonberry leaves and fruit, respectively). Lower amounts of remaining monomeric flavanols and flavonol aglycones - quercetin, kaempferol - were detected; the total content of all identified compounds in the acetone leaves fraction was 95.7 mg/g and in the fruit fraction was 40.5 mg/g.

### Anticancer activity

### Cell cultures

The human colorectal cancer cell line HT-29, the human malignant melanoma cell line IGR39, and the human renal adenocarcinoma cell line CaKi-1 were obtained from an American-type culture collection (ATCC, Manassas, VA, USA). Cells were grown in DMEM Glutamax medium (Gibco, Carlsbad, CA, USA) containing 10% fetal calf serum and 1% antibiotic mixture (10,000 U/ml penicillin and 10 mg/ml streptomycin; Gibco). All cells were incubated at 37° C in a humidified atmosphere containing 5% CO₂ till passage 20. Selected cell cultures are of human colorectal, renal, and melanoma cells.

### Cell migration study and spheroid preparation

The effect of lingonberry fruit and leaves proanthocyanidin fractions on cell migration was determined using the "wound healing" method. IGR-39, CaKi-1, and HT-29 cells were seeded in a 24-well plate and grown for 1-2 days at 37°C in a humidified atmosphere containing 5% CO₂ until a monolayer was formed. Then, with 100 µl pipette tip, sweep vertically through the cells in each well and make a scratch - the so-called "wound". The medium is aspirated from the cells, washed with PBS solution, and the medium with different concentrations of test extracts is added. The concentrations of lingonberry leaf extract from 10 µg/ml to 100 µg/ml and of lingonberry fruit extract from 5 µg/ml to 100 µg/ml were chosen for these cell lines during the MTT assay. Three wells are used for each concentration. Wells containing cells without extracts, with nutrient medium only, are used as negative controls. Cells were incubated at 37 ºC during the experiment, 5% CO₂ in the atmosphere. The change in the area of the wound is assessed after 24 and 48 hours. (IGR39 cell migration experiments only) or after 12, 24, and 36 h. from the start of the experiment (CaKi-1 and HT-29 cell migration experiments) until the wound "healed" in the control group (Fig. 2). Wells are photographed under a light microscope, images are processed with imaged software, and the percentage change in area is estimated.

### Study of effects on spheroids

Spheroids were prepared from IGR39, HT-29, and CaKi-1 cell lines using the magnetic 3D Bioprinting method [Souza GR., Et al., 2010]. The cancer cells are incubated in a plate of 6 wells so that there are about 200,000-300,000 cells in one well. The plate is left for 12-14 hours in an incubator at 37°C and 5% CO₂ in the atmosphere. After incubation, 20 µl of NanoShuttle nanoparticles are added to each well and incubated for 8 hours. After incubation, the medium is aspirated, the cells are washed with PBS, trypsinized, and cell suspensions are prepared in cell culture medium. The suspensions are prepared so that 100 µl of cancer cells are present in each well of the plate when dispensed into ultra-low attachment 96-well plate. The plate thus prepared is placed on a magnetic plate and left in an incubator at 37°C and 5% CO₂ concentration. Incubate for 2-3 days until spheroids form. The culture medium is then replaced with a fresh medium in which the lingonberry leaves and fruit proanthocyanidin fractions are dissolved. The concentrations of lingonberry leaf extract from 10 µg/ml to 100 µg/ml and of lingonberry fruit extract from 5 µg/ml to 100 µg/ml were chosen for these cell lines during the MTT assay. Spheroids are photographed under a phase contrast microscope using 4× magnification lens. The spheroids are then photographed every other day, and the medium is changed every other day as well. The culture medium is replaced by a fresh medium with the same concentrations of proanthocyanidin fractions. Negative control - spheroids with medium only (no proanthocyanidin fractions). After the first photograph, the plate with the already formed spheroids is incubated without a magnetic plate at 37ºC and 5% CO₂ in the atmosphere. The effect of extracts on their growth is evaluated by the change in the size of the spheroids. The analysis is performed by the imaged program (National Institutes of Health, USA).

On the last day of the experiment (after 10 days of incubation with proanthocyanidin fractions), the viability of the cells in the spheroids was examined by the MTT assay. The medium is aspirated from the spheroids, and 100 µL of fresh medium containing 10 µL of MTT (5 mg / ml) is added and incubated for 10-12 h. The medium is then discarded, and the formed formazan crystals are dissolved in 100 µl of DMSO (incubated for 5-6 hours). Absorbance was measured at 570 nm and 630 nm, and cell viability was calculated as a percentage of control. Wells free of spheroids (medium with MTT reagent only) were used as controls.

### Results

The prepared lingonberry leaves and fruit proanthocyanidin fractions are used for the assessing the property of a cell selected from any of formation, growth, proliferation, migration, viability, disintegration.

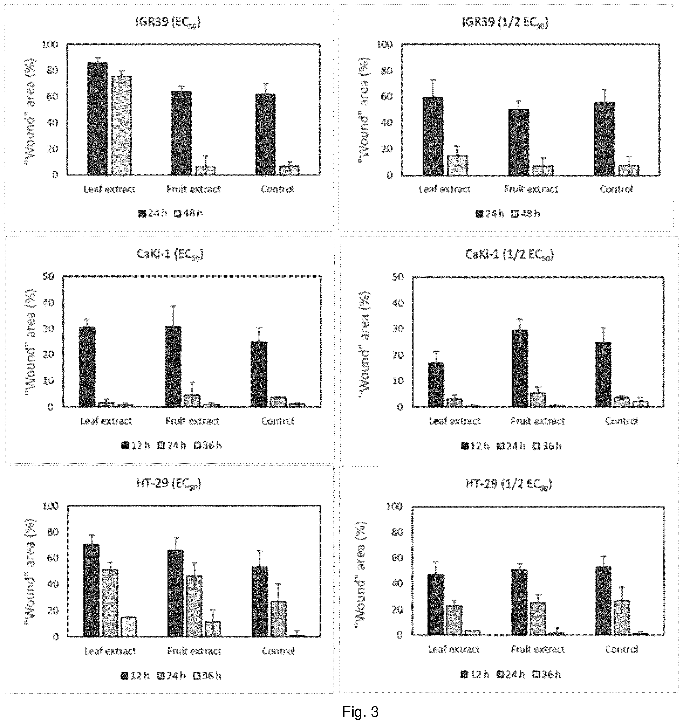

The lingonberry leaves and fruit proanthocyanidin fractions tested showed an inhibitory effect on IGR39, CaKi-1 and HT-29 cell migration by the "wound healing" method (Fig. 3). The most active was the leaves extract, especially the concentration corresponding to 5 µg/ml to 100 µg/ml. After 48 hours, the area of the "wound" of the incubation in terms of the effect on IGR39 cell migration was 75.3 ± 8.5%, while in the control group the "wound" was reduced to 6.7 ± 5.5%. A strong antimigrant effect of this extract on HT-29 cells was also found. After 36 hours incubation resulted in an approximately 15-fold larger area of unoccupied "wound" compared to the control group.

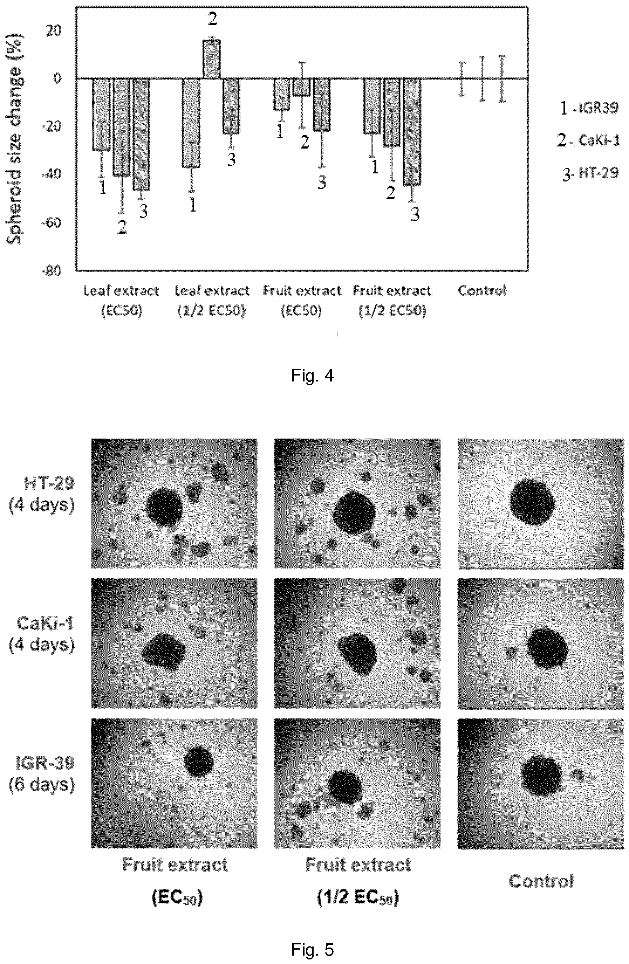

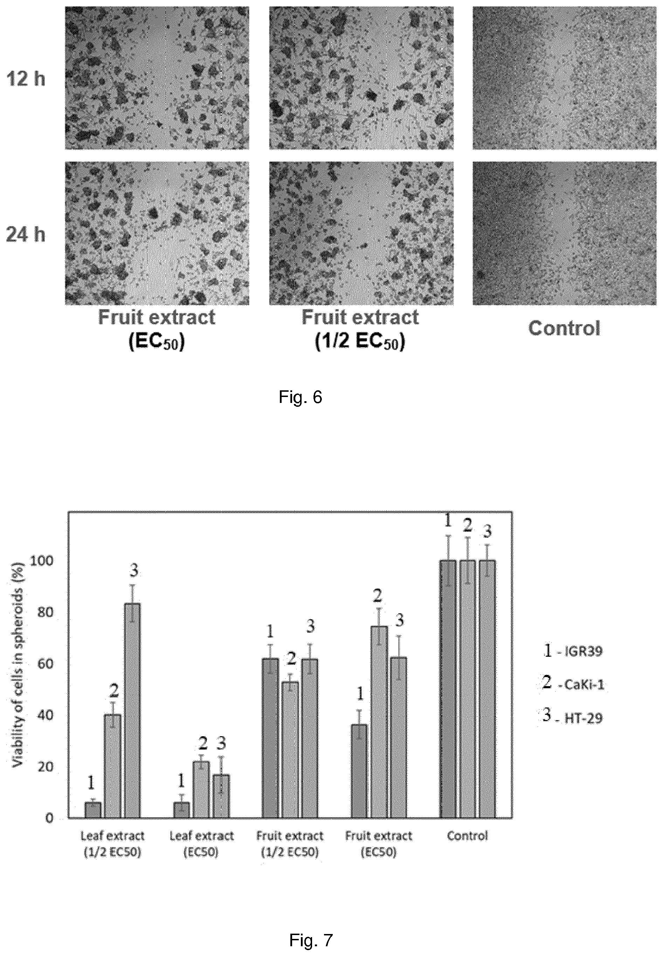

Lingonberry leaves and fruit proanthocyanidin fractions inhibited the growth of spheroids from IGR39, CaKi-1, and HT-29 cells, as measured by changes in spheroid area (Fig. 4). The most active was the leaves extract used at 5 µg/ml to 100 µg/ml concentrations as well as the fruit extract used at 10 µg/ml to 100 µg/ml concentration. All spheroids, especially HT-29, grew more slowly when exposed to these proanthocyanidin fractions, and their area after 10 days of incubation was about 40% smaller compared to the control group (spheroids unaffected by the extracts). Fruit proanthocyanidin fraction used at 10 µg/ml to 100 µg/ml concentration promoted spheroid formation after 4 or 6 days of incubation, especially HT 29 spheroid formation (Fig. 5). The spheroid-inducing effect was very strong in conventional plates by incubating monolayer-growing HT-29 cells with 10 µg/ml to 100 µg/ml of lingonberry fruit proanthocyanidin fraction concentrations (Fig. 6). In this case, spontaneous formation of HT-29 spheroids was observed after 12 hours of incubation. After 24 hours, almost all cells were in spheroids, which kept growing for another 24 hours of the experiment.

Evaluation of cell viability in spheroids by MTT assay showed a decrease in spheroid viability under the influence of lingonberry leaves and fruit proanthocyanidin fractions (Fig. 7). The greatest antiproliferative effect was seen on IGR39 cell spheroids, especially when treated with leaves proanthocyanidin fraction (their viability was 6-6.1%). Also, a strong effect of higher concentrations of lingonberry leaves proanthocyanidin fraction corresponding to the EC₅₀ value on spheroid cell viability was observed for HT-29 spheroids (viability was 16.8 ± 7.1%) and CaKi-1 spheroids (viability was 21.8 ± 2.6 %).

The prepared extracts are for use in the treatment of cancers selected from the group consisting of brain, head and neck, lung, breast, stomach, liver, pancreas, prostate, uterine, colorectal, renal and melanoma cancers. The proanthocyanidin fractions in particular are suitable for the treatment of colorectal, renal and melanoma cancers.

## Claims

1. A method for the preparation of cell spheroid comprising contacting a cell suspension with the lingonberry proanthocyanidin fractions, wherein the lingonberry proanthocyanidin fractions are dissolved in water.

2. The method according to claims 1, wherein cell spheroid comprises cancer cells.

3. The method according to claim 2, wherein the cell spheroid comprises colorectal cancer cell line, the human malignant melanoma cell line, and the human renal adenocarcinoma cell line.

4. The method according to any of claims 1-3, wherein the method comprises producing cell spheroids by contacting a cell line with lingonberry proanthocyanidin fractions.

5. The method according to any of claims 1-4, wherein the lingonberry proanthocyanidin fractions are obtained from lingonberry leaves extracts.

6. The method according to any of claims 1-5, wherein the concentration of lingonberry leaves extract is from 5 µg/ml to 100 µg/ml.

7. The method according to any of claims 1-4, wherein the lingonberry proanthocyanidin fractions are obtained from lingonberry fruit extracts.

8. The method according to any of claims 1-5 and 7, wherein the concentration of lingonberry fruit extract is from 10 µg/ml to 100 µg/ml.

9. The method according to any of claims 1-8, wherein the lingonberry proanthocyanidin fractions are in a cell culture plate.

10. The lingoberry proanthocyanidin fraction comprising cell spheroids, wherein cell spheroids are cancer spheroids.

11. Use of the lingoberry proanthocyanidin fraction according to claim 10 in assessing the property of a cell selected from any of formation, growth, proliferation, migration, viability, disintegration.

12. The lingonberry proanthocyanin fraction according to claim 10 for use in the prevention or treatment of a cancerous condition, which is selected from the group consisting of colorectal cancer, kidney cancer, and melanoma.

13. The lingonberry proanthocyanin fraction, for use according to claim 12, wherein the cancerous condition involves metastasis that displays accelerated cell migration.
